# EUROPEAN PATENT APPLICATION

(11) **EP 2 243 780 A2**
(43) Date of publication of application: **27.10.2010**
(21) Application number: 10157328.5
(22) Date of filing: 23.03.2010
(51) Int. Cl.: C07D 471/04

(54) **Process for the purification of paliperidone**

(30) Priority: 21.04.2009 IT MI20090663
(71) Applicant: Dipharma Francis S.r.l., 20021 Baranzate (MI) (IT)
(72) Inventor: Mantegazza, Simone, 20144, MILANO (IT); Attolino, Emanuele, 74019, PALAGIANO (TA) (IT); Razzetti, Gabriele, 20099, SESTO SAN GIOVANNI (MI) (IT); Allegrini, Pietro, 20097, S. DONATO MILANESE (MI) (IT)
(74) Representative: Minoja, Fabrizio

(57) **Abstract**

Process for the purification of paliperidone by formation of a salt thereof, such as the hydrochloride.

## Description

### FIELD OF INVENTION

The present invention relates to a process for the purification of paliperidone by using a pharmaceutically acceptable salt thereof, such as the hydrochloride salt.

### PRIOR ART

Paliperidone, namely (±)-3-[2-[4-(6-fluoro-1,2-benzoisoxazol-3-yl)-1-piperidinyl]ethyl-6,7,8,9-tetrahydro-9-hydroxy-2-methylpropanediol-4H-pyrido[1,2-a]pyrimidin-4-one, having the following formula (I) 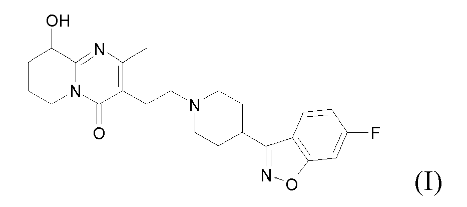 is a 5-HT antagonist belonging to the class of benzisoxazoles, and is used in the treatment of schizophrenia. Paliperidone is known from US 5,158,952, which discloses its preparation by reaction of 3-(chloroethyl)-6,7,8,9-tetrahydro-9-hydroxy-2-methyl-4H-pyrido[1,2-a]-pyrimidin-4-one of formula **(II)** with 6-fluoro-3-(4-piperidinyl)-1,2-benzoisoxazole of formula **(III)** in a solvent, in the presence of a suitable base, according to the following scheme:

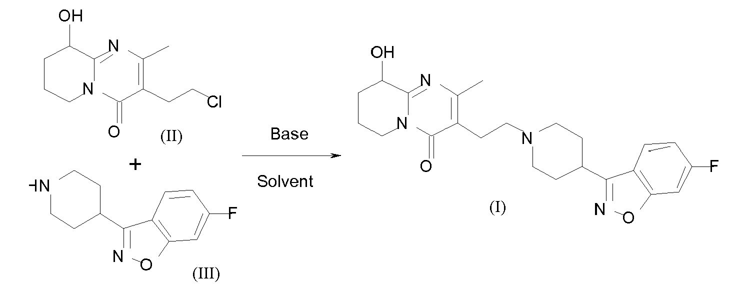

Paliperidone as free base, obtained according to the preparation procedures exemplified in US 5,158,952, has very low chemical yield and HPLC purity. Therefore, said process is not well-suited to the production on an industrial scale. The known preparation processes usually afford paliperidone admixed with impurities, such as paliperidone N-oxide of formula **(IV)** and the carbamate of formula **(V).** WO 2008/021346, which discloses a process for the purification of paliperidone as the free base, discloses these impurities, which are formed in variable amounts according to the reaction conditions and, in the case of the N-oxide of formula (IV), also according to the conditions in which paliperidone is isolated from the reaction mixture.

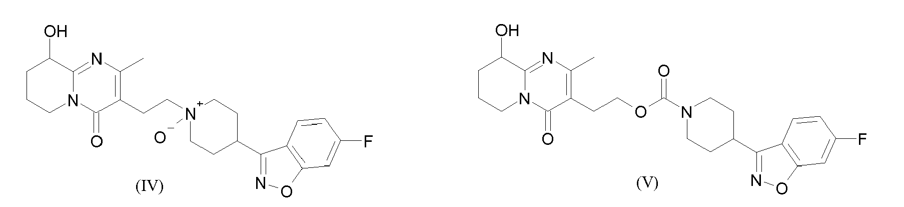

As it is known, the efficiency of purification depend on the crystalline structure of the starting and ending paliperidone polymorph; the solubility of paliperidone in the different mixtures of solvents used for its purification varies indeed with that structure.

As shown on page 2/58 of the documentation present on the EMEA (European Medicines Agency) website (http://www.emea.europa.eu/humandocs/PDFs/EPAR/invega/H-746-en6.pdf) relating to paliperidone, there are two polymorphic crystalline forms of paliperidone named Forms I and II, in addition to a hydrate form and a solvate form, Form I being the most thermodynamically stable. The large number of solid polymorphic forms of paliperidone, which often crystallise as a mixture of crystalline forms, makes its purification as free base complex and industrially unfeasible, using known techniques. A simple, efficient method for the purification of paliperidone, which is feasible on an industrial scale, is therefore required.

### SUMMARY OF THE INVENTION

A process for the purification of paliperidone by means of the preparation thereof as a hydrated base and the subsequent conversion thereof to the known Form I has now been found.

### BRIEF DESCRIPTION OF FIGURES AND ANALYSIS METHODS

The crystalline forms of paliperidone and paliperidone hydrochloride have been characterised by potentiometric and argentimetric titration, X-ray powder diffraction (XRPD), ¹H-NMR Nuclear Magnetic Resonance spectrometry, and differential scanning calorimetry (DSC). The water content of the compounds was determined by titration with the Karl Fischer technique. The X-ray diffraction spectrum (XRPD) was collected with the APD-2000 automatic θ/θ powder and liquid diffractometer made by Ital-Structures, under the following operating conditions: CuKα radiation (λ = 1.5418 Å), scanning with a 2θ angle range of 3-40° and a step size of 0.03°, for a time of 1 second. The ¹H-NMR spectra were acquired with a Varian Mercury 300 spectrometer, using DMSO-d6 as solvent. The DSC thermograms were acquired with a Mettler-Toledo DSC 822e differential scanning calorimeter, under the following operating conditions: aluminium capsules, range 30-300°C at the rate of 10°C/min, with nitrogen as purge gas (80 ml/min). The particle size was determined by the well-known laser light scattering technique, using a Malvern Mastersizer MS1 instrument under the following operating conditions:
- 300RF mm lens with 2.4 mm laser beam length;
- 500 mg sample dispersed in 10 ml of hexane (ACS reagent) with 1% of SPAN 85^{®}, without pre-sonication, and with a stirring rate of 2500 rpm.

Figure 1: XRPD spectrum of paliperidone hydrochloride 1:1 (paliperidone:acid).
Figure 2: DSC thermogram of paliperidone hydrochloride 1:1 (paliperidone:acid).
Figure 3: XRPD spectrum of paliperidone base hydrate.
Figure 4: DSC thermogram of paliperidone base hydrate.
Figure 5: XRPD spectrum of paliperidone crystalline Form I.
Figure 6: DSC thermogram of paliperidone crystalline Form I.

### DETAILED DESCRIPTION OF THE INVENTION

The present invention relates to a process for purifying paliperidone, for example crude paliperidone, and obtaining it in hydrated form, which comprises:
a) forming a paliperidone 1:1 (paliperidone:acid) addition salt with a strong protic acid in water;
b) recovering the so obtained solid;
c) forming a dispersion of the so obtained paliperidone 1:1 (paliperidone:acid) addition salt in an aqueous solvent;
d) treating said dispersion with a base; and
e) recovering the so obtained paliperidone base hydrate.

Paliperidone, used as starting material, can be a crude paliperidone as obtainable according to US 5,158,952.

A paliperidone addition salt in accordance with step a) may be, for example, an addition salt with a strong protic acid, preferably pharmaceutically acceptable, typically a hydrohalic acid, preferably hydrochloric acid; or sulphuric acid or a sulphonic acid, preferably para-toluenesulphonic acid.

Most preferably, the addition salt is paliperidone hydrochloride.

A paliperidone addition salt can be obtained, for example, by treating a suspension of crude paliperidone in water with a concentrated aqueous solution of an acid, as defined above.

The so obtained solid paliperidone addition salt can be recovered using known techniques such as filtration or centrifugation, optionally followed by drying, preferably under vacuum. The product is preferably recovered by filtration through a Bückner filter, followed by drying under vacuum at a temperature between about room temperature and about 60°C, preferably at about 45-55°C, and more preferably at about 50°C.

The solid paliperidone addition salt can optionally be purified by subjecting it to one or more recrystallisations, preferably one or two, from water or from a mixture comprising water and at least one solvent, preferably one or two, selected from the group comprising a C₃-C₆ ketone, typically acetone or methyl ethyl ketone, more preferably acetone; a C₁-C₆ alkanol, such as methanol, ethanol or isopropanol; an acyclic ether, such as diethyl ether; and a cyclic ether, such as tetrahydrofuran or dioxane; until a paliperidone salt with a purity degree equal to or higher than 99%, in particular equal to or higher than 99.5%, and more preferably equal to or higher than 99.9%, is obtained.

The recrystallisation can be carried out, for example, by heating the mixture to a temperature between about 70°C and the solvent boiling point, preferably about 90°C, and then cooling the reaction mixture to a temperature between about 0 and 20°C, preferably about 0-5°C.

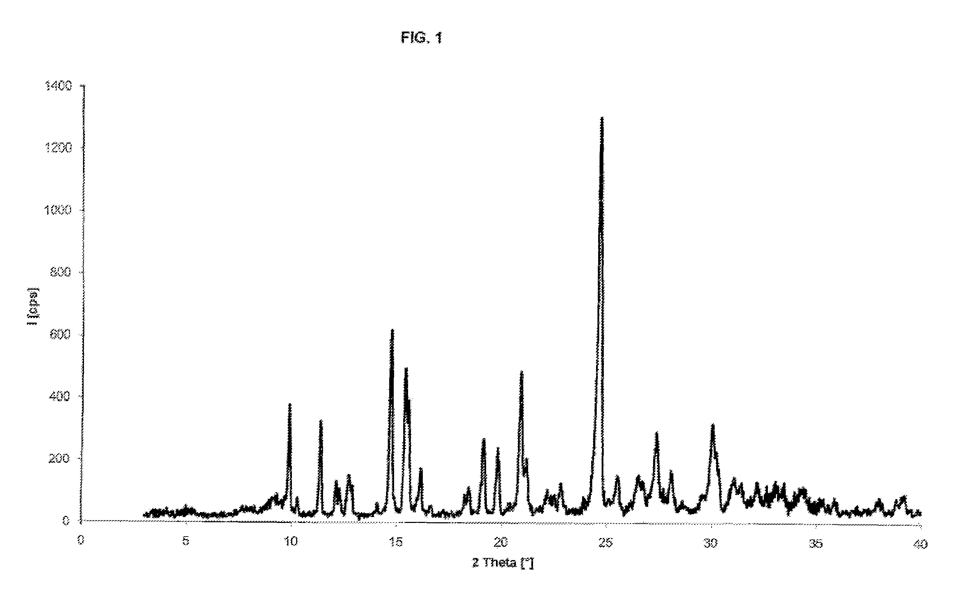

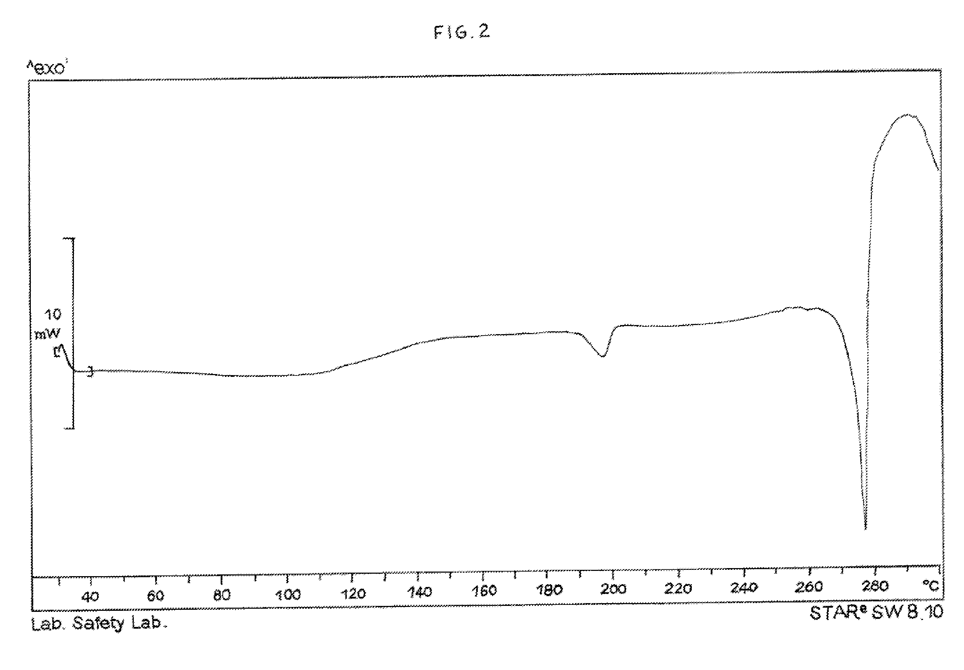

When the strong protic acid in step a) is hydrochloric acid, the paliperidone hydrochloride thus obtained is a crystalline solid poorly soluble in water, characterised by a DSC thermogram as shown in Figure 2, with a melting peak at 276 ± 2°C and an XRPD spectrum as illustrated in Figure 1, wherein the most intense diffraction peaks fall at 9.9; 10.3; 11.4; 12.1; 12.7; 14.7; 15.4; 16.1; 19.1; 19.8; 20.9; 24.7; 27.4 ± 0.2° in 2θ.

The particle size of the thus obtained paliperidone hydrochloride crystals is characterised by a value of D₅₀ between about 25 and 250 µm, wherein D₅₀ is the particle diameter such that 50% (in volume) of the particle sample has a diameter equal to or lower than the specific value. If desired, said value can be reduced by micronisation or fine grinding.

According to the potentiometric acid-base and argentimetric titrations, the paliperidone hydrochloride thus obtained is a 1:1 addition salt of paliperidone with an equivalent of hydrochloric acid (paliperidone monohydrochloride). The titre of the chlorides is 7.8% (theoretical value of monohydrochloride 7.6 - 8.0%). Analogously, its potentiometric titration with sodium hydroxide indicates a point of equivalence and stoichiometric consumption of sodium hydroxide.

Said crystalline form of paliperidone hydrochloride is novel, and is a further object of the present invention.

A dispersion of a 1:1 addition salt of paliperidone in an aqueous solvent, in accordance with step c), can be prepared by suspending the solid previously obtained in an aqueous solvent consisting of a mixture containing water and one or more, preferably one or two, water-miscible solvents, independently selected from, for example, a C₃-C₅ ketone, such as acetone or methyl ethyl ketone and a C₁-C₅ alkanol, such as methanol or isopropanol, preferably isopropanol. Said mixture is hereinafter called "aqueous solvent".

According to a preferred aspect of the invention, the paliperidone purification process reported above in step c) can also include treatment of the 1:1 addition salt with a strong protic acid to obtain a solution of the 1:2 (paliperidone:acid) addition salt.

A solution of the 1: 2 addition salt [paliperidone:acid] can be obtained, for example, by treating the water dispersion of the 1:1 (paliperidone:acid) addition salt with a strong protic acid as defined above. The same acid as used to form the 1:1 (paliperidone:acid) addition salt is preferably used.

Said dispersion of the 1:1 or 1:2 addition salt is then treated with a base in accordance with step d). A base may be, for example, an inorganic base, typically an alkaline metal hydroxide, such as sodium hydroxide or potassium hydroxide; an alkaline earth metal hydroxide, such as calcium hydroxide or barium hydroxide, more preferably sodium hydroxide, potassium hydroxide or barium hydroxide; or a C₁-C₆ alkoxide, such as sodium or potassium ethoxide, or tert-butoxide; or an organic base, such as a tertiary amine, for example triethylamine or diisopropylethylamine. More preferably the base is sodium hydroxide or potassium hydroxide.

The solid paliperidone hydrate thus obtained can be recovered by known techniques such as filtration or centrifugation, optionally followed by drying, preferably under vacuum. The product is preferably recovered by filtration, followed by drying under vacuum at a temperature between about room temperature and about 60°C, preferably at about 45-55°C, and more preferably at about 50°C.

The solid paliperidone base hydrate thus obtained has a water content of between about 7.0 and about 8%, preferably around 7.3 - 7.5%, so that it can be defined about as a dihydrate.

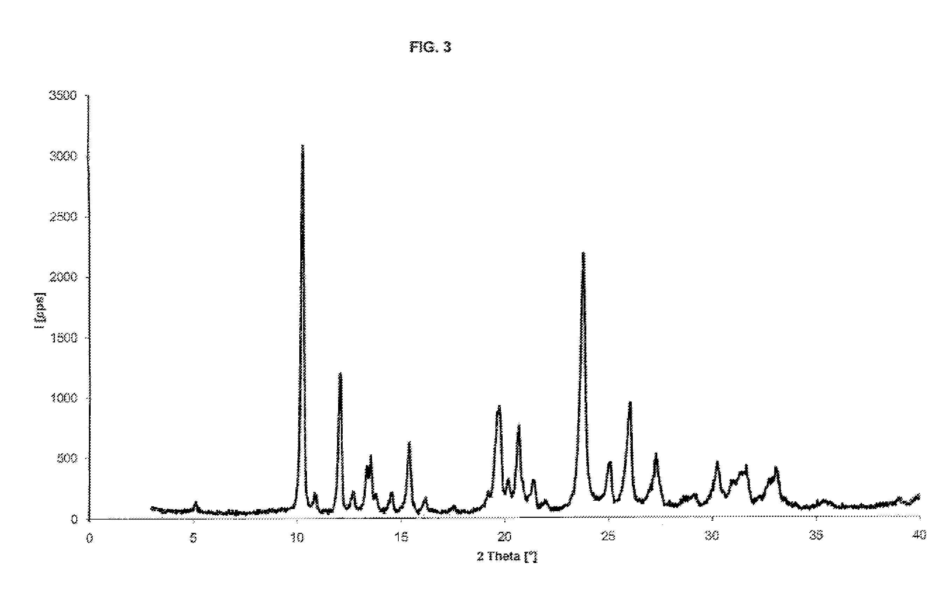

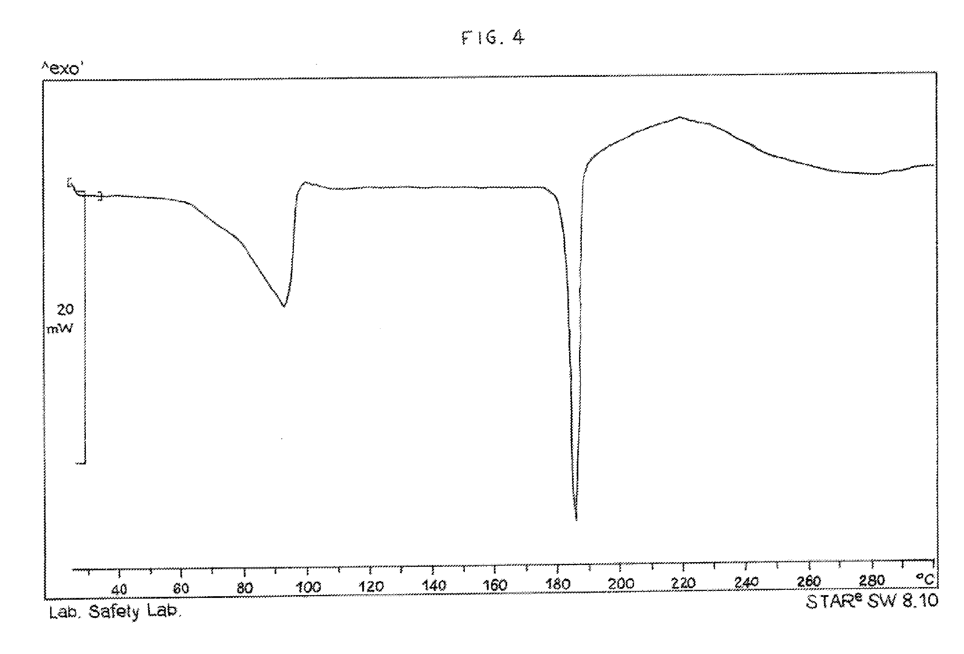

The paliperidone base hydrate thus obtained is characterised by a DSC thermogram as shown in Figure 4, with a melting peak at 182.5 ± 2°C. Said hydrated form has an XRPD spectrum as shown in Figure 3, where the most intense diffraction peaks fall at 5.1; 10.3; 10.9; 12.1; 12.7; 13.6; 14.6; 15.4; 16.2; 19.7; 20.7; 23.8; 25.1; 26.0; 27.3 ± 0.2° in 2θ, and has a degree of purity substantially equal to that of the addition salt from which it derives, so that it meets the regulatory requirements for pharmaceutical products.

If desired, the thus obtained paliperidone base hydrate can be converted to another crystalline form, such as the more stable anhydrous crystalline polymorph of Form I, as obtainable according to example 4 in US 5,158,952.

According to a further aspect of the invention, the paliperidone base hydrate can be converted into paliperidone anhydrous crystalline Form I by a process comprising:
- dissolving paliperidone hydrate in an aqueous solvent as defined above, or in an organic solvent selected from a straight or branched C₃-C₈ ketone, such as acetone or methyl ethyl ketone, and a C₁-C₅ alkanol, preferably isopropanol; at a temperature between about 70°C and the solvent boiling point, preferably at about 90°C;
- subsequent cooling of the reaction mixture to a temperature between about 0 and 20°C, preferably about 0-5°C; and
- recovering the so obtained solid.

More preferably, said solvent is a water/acetone mixture or acetone.

The solid can be recovered by known methods and dried at a temperature between room temperature and about 60°C, preferably at about 45-55°C, and more preferably at about 50°C.

A further object of the present invention is a process for the preparation of anhydrous paliperidone crystalline Form I, which comprises:
- forming a solution of a 1:2 [paliperidone:acid] addition salt with a strong protic acid in water;
- treating said solution with a base dissolved in an organic solvent selected from a C₁-C₅ alkanol and a straight or branched C₃-C₈ ketone;
- recovering the so obtained anhydrous paliperidone crystalline Form I.

A solution of a 1:2 [paliperidone:acid] addition salt with a strong protic acid as defined above can be formed, for example, as reported above.

The base may be, for example, an organic or inorganic base as defined above, preferably triethylamine, sodium hydroxide, potassium hydroxide, sodium bicarbonate, potassium bicarbonate or ammonium bicarbonate, more preferably triethylamine.

The organic solvent is preferably a C₁-C₅ alkanol, more preferably methanol.

The solution can be treated with a base at a temperature between about 0°C and about 30°C, preferably between about 20 and 30°C; followed by cooling of the reaction mixture to a temperature between about 0 and 20°C, preferably about 0-5°C.

The anhydrous paliperidone crystalline Form I can be recovered using known methods, such as filtration and subsequent drying at a temperature between room temperature and about 60°C, preferably at about 45-55°C, and more preferably at about 50°C.

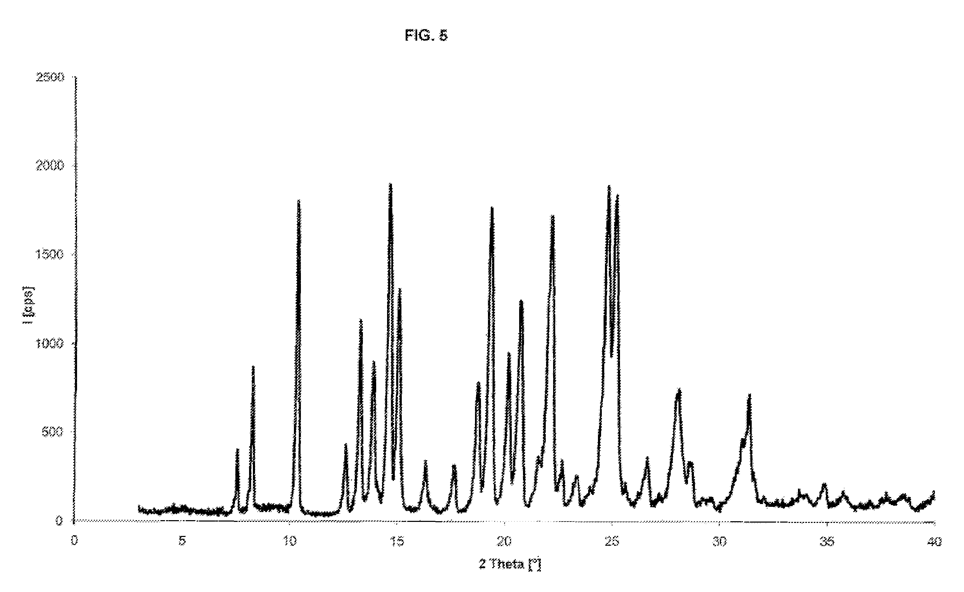

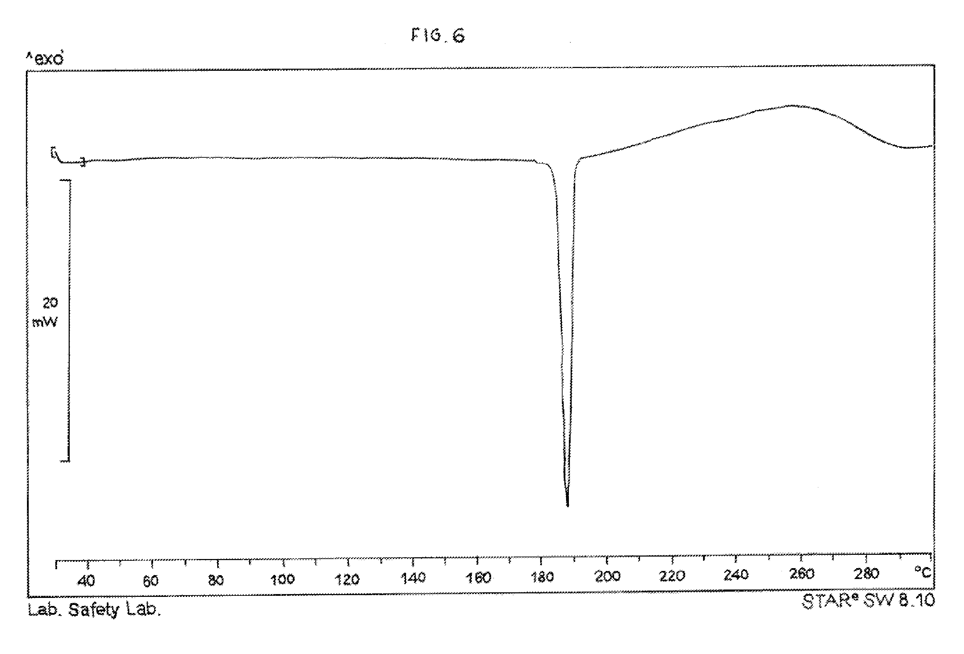

Polymorphic paliperidone Form I is a crystalline solid poorly soluble in water, characterised by a DSC thermogram as shown in Figure 6, with a melting peak at 187 ± 2°C, and an XRPD spectrum as illustrated in Figure 5, wherein the most intense diffraction peaks fall at 7.6; 8.3; 10.4; 12.6; 13.3; 13.9; 14.6; 15.1; 16.3; 17.6; 18.7; 19.4; 20.2; 20.7; 22.2; 24.8; 25.2 ± 0.2° in 2θ.

The size of the crystalline polymorphic paliperidone crystals thus obtained is characterised by a value of D₅₀ between about 25 and 1000 µm, wherein D₅₀ indicates a particle diameter such that 50% (in volume) of the particle sample has a diameter equal to or lower than the specific value. If desired, said value can be reduced by micronisation or fine grinding.

The thus obtained crystalline polymorphic paliperidone Form I has a purity equal to or higher than 99.5%, more preferably equal to or higher than 99.9%.

A further object of the invention is a pharmaceutical composition comprising paliperidone hydrochloride, having a DSC thermogram as shown in Figure 2, and an XRPD spectrum as illustrated in Figure 1, as active ingredient, and a pharmaceutically acceptable excipient and/or vehicle. Said pharmaceutical composition can be prepared by known methods.

According to a preferred aspect of the invention, paliperidone, for example as the crude product, can be obtained through the route of synthesis disclosed in US 5,158,952, but using methanol as solvent, and with the reaction conducted in the presence of triethylamine. In fact, it has surprisingly been found that the use of triethylamine as base in the alkylation reaction of the compound of formula **(III)** with the compound of formula **(II)** produces a crude paliperidone, isolated by evaporation of the reaction solvent under reduced pressure, with a yield equal to or higher than 95%, preferably equal to or higher than 96.5% (HPLC titre).

A further object of the present invention is therefore a process for the preparation of paliperidone comprising alkylation of a compound of formula **(III),** as defined herein, with a compound of formula **(II),** as defined herein, in methanol and in the presence of triethylamine.

The following examples illustrate the invention.

### Example 1 - Synthesis of 3-[2-[4-(6-fluoro-1,2-benzoisoxazol-3-yl)-1-piperidinyl]ethyl-6,7,8,9-tetrahydro-9-hydroxy-2-methyl-4H-pyrido[1,2-a]-pyrimidin-4-one hydrochloride (Paliperidone hydrochloride)

6-Fluoro-3-(4-piperidinyl)-1,2-benzoisoxazole hydrochloride (300 g, 1.17 mols), 3-(chloroethyl)-6,7,8,9-tetrahydro-9-hydroxy-2-methyl-4H-pyrido[1,2-a]pyrimidin-4-one (343 g, 1.40 mols) and triethylamine (272 g, 2.69 mols) are suspended in methanol (1.5 1) in a 3000 ml reactor under nitrogen atmosphere, and the reaction mixture is heated at reflux temperature for 18-20 h. Conversion to the product is checked by HPLC titre, obtaining a yield of 96.5% in solution. The mixture is concentrated to a residue. The so obtained product has an XRPD spectrum as shown in Figure 5, and a DSC thermogram as shown in Figure 6, which are characteristic of paliperidone crystalline Form I.

The mixture is taken up with demineralised water (1.5 1) and 36.5% hydrochloric acid (113 g, 1.13 mols), obtaining a solution with a pH of 3-4. A solid then reprecipitates, and the mixture is cooled to 0°C and filtered. The solid is washed with demineralised water cooled to 0-5°C (2 x 150 ml), and then with acetone cooled to 0-5°C (3 x 200 ml). The solid is dried in oven under reduced pressure at a temperature of 50°C for 16-18h. 451 g of paliperidone hydrochloride is obtained, with a potentiometric titre of 99.9%, an argentimetric titre of 7.8%, 99.2% HPLC purity, and a total yield of 90%. The product has an XRPD spectrum as shown in Figure 1, and a DSC thermogram as shown in Figure 2.

### Example 2 - Purification of Paliperidone hydrochloride

Paliperidone hydrochloride obtained, for example, analogously to the procedure disclosed in example 1 (20.0 g, 43.2 mmols), having 98.8% HPLC purity, is suspended in a solvent mixture consisting of acetone (30 ml) and demineralised water (30 ml) at room temperature in a 250 ml flask under inert atmosphere. The mixture is heated to boiling point, keeping this temperature until the solid is completely dissolved. The mixture is then left to cool slowly to room temperature, and then to 0-5°C for at least 1h. The suspended solid is filtered and washed with acetone cooled to the temperature of 0-5°C (3 x 10 ml), and then dried in oven overnight at 50°C. 18.0 g of the product is obtained, with 99.7% HPLC purity and a 90% crystallisation yield. The product has an XRPD spectrum as shown in Figure 1, and a DSC thermogram as shown in Figure 2.

### Example 3 - Purification of Paliperidone hydrochloride

Paliperidone hydrochloride obtained, for example, in a similar way to the procedure disclosed in example 1 (38.3 g, 82.7 mmols), with 99.4% HPLC purity, is added to demineralised water (110 ml) in a 250 ml flask under inert atmosphere, and the mixture is heated to the temperature of about 90°C, keeping this temperature until the solvent is completely dissolved. The mixture is then left to cool at room temperature in about 3 h, and further cooled to about 0-5°C for at least 1 h. The solid is recovered by filtration and washed with water cooled to 0-5°C (2 x 25 ml). After drying in oven at the temperature of 50°C, 36.7 g of the product is obtained, with 99.9% HPLC purity and a 96% yield. The product has an XRPD spectrum as shown in Figure 1, and a DSC thermogram as shown in Figure 2.

### Example 4 - Precipitation of Paliperidone base hydrate

Paliperidone hydrochloride, as obtainable from examples 1, 2 or 3 (300 g, 0.620 mol) is added to a 36.5% solution of hydrochloric acid (68.1 g, 0.681 mol) in demineralised water (900 ml) in a 2000 ml flask maintained under nitrogen, at room temperature. Decolourising charcoal (15 g) is added to the so obtained solution, which is then left under stirring for 15-20 minutes. Charcoal is removed by filtration through a perlite panel. The clarified solution is slowly added to a 3000 ml reactor containing isopropyl alcohol (1200 ml) and a 30% solution of sodium hydroxide in water (250 g, 1.86 mols). The temperature is maintained under 10°C during the addition; the formation of a precipitate is observed. After completion of the addition, the mixture is kept cold for about 1 h and then filtered. The solid is washed with a 1:1 mixture of water/isopropanol (3 x 150 ml) and then dried in oven overnight at the temperature of 50°C. 258 g of the product is obtained, with a potentiometric titre of 94.7% and KF 7.5%, 99.9% HPLC purity and a 93% precipitation yield. The product has an XRPD spectrum as shown in Figure 3, and a DSC thermogram as shown in Figure 4.

### Example 5 - Conversion of Paliperidone hydrate to Paliperidone crystalline Form I

Paliperidone base hydrate, as obtained in example 4, is suspended in a solvent mixture consisting of acetone (6.0 l) and demineralised water (2.0 l) in a 10 litre reactor maintained in an inert atmosphere. The mixture is heated to boiling point until the solid has completely dissolved, and then left to cool spontaneously overnight to room temperature. The mixture is cooled to the temperature of 0-5°C for at least 2 h, and the solid is filtered and washed with acetone cooled to 0-5°C (3 x 250 ml). The solid is dried in oven at the temperature of 50°C under reduced pressure, and 180 g of the product is obtained, with a potentiometric titre of 99.6%, KF 0.04%, and 99.9% HPLC purity. Crystallisation yield 92%. The product has an XRPD spectrum as shown in Figure 5, and a DSC thermogram as shown in Figure 6.

### Example 6 - Precipitation of crystalline Paliperidone Form I

Paliperidone hydrochloride (30 g, 0.062 mol) is added to a 36.5% solution of hydrochloric acid (6.8 g, 0.068 mol) in demineralised water (150 ml) in a flask maintained under inert atmosphere, at room temperature. Decolourising charcoal (1.5 g) is added to the so obtained solution, which is then left under stirring for 15-20 minutes. Charcoal is removed by filtration through a perlite panel. The clarified solution is added slowly to a solution of triethylamine (19 g, 0.19 mol) in methanol (150 ml). During addition the temperature is maintained at under 30°C, and the formation of a precipitate is observed. After completion of the addition, the mixture is cooled for about 1 h to the temperature of 0-5°C, and filtered. The solid is washed with a 1/1 mixture of water/methanol (3 x 15 ml), and then dried in oven overnight at the temperature of 50°C. 25 g of the product is obtained, with a potentiometric titre of 99.5%, KF 0.06%, and 99.9% HPLC purity. The product has an XRPD spectrum as shown in Figure 5, and a DSC thermogram as shown in Figure 6.

### Example 7 - Preparation of Paliperidone crystalline Form I

Paliperidone hydrate with KF 7.3% (2.7 kg, 5.8 mols) is suspended in acetone (110 l), in a 200 litre reactor rendered inert with nitrogen, and the mixture is heated at reflux temperature for 2 hours. The mixture is cooled in about 3.5 hours to the temperature of 0°C. The solid is filtered, and washed with acetone cooled to 0-5°C (2 x 2.5 l). The product is dried at 50°C under vacuum for 18-20 hours. 2.5 kg of the product is obtained, with a 96% yield, potentiometric titre of 99.8%, KF 0.05%, and 99.9% HPLC purity. The product has an XRPD spectrum as shown in Figure 5, and a DSC thermogram as shown in Figure 6.

## Claims

1. A process for purifying paliperidone and obtaining it in hydrated form, which comprises:
a) forming a paliperidone 1:1 (paliperidone:acid) addition salt with a strong protic acid in water;
b) recovering the so obtained solid;
c) forming a dispersion of the so obtained paliperidone 1:1 (paliperidone:acid) addition salt in an aqueous solvent;
d) treating said dispersion with a base; and
e) recovering the so obtained paliperidone base hydrate.

2. The process according to claim 1, wherein the strong protic acid is selected from the group consisting of a hydrohalic acid, sulphuric acid and a sulphonic acid, preferably hydrochloric acid.

3. The process according to claim 1, wherein the aqueous solvent is a mixture comprising water and one or more, preferably one or two, water-miscible solvents, preferably a C₃-C₅ ketone or a C₁-C₅ alkanol.

4. The process according to claim 3, wherein the C₁-C₅ alkanol is isopropanol.

5. The process according to claim 1, wherein step c) further comprises the treatment of the 1:1 addition salt with a strong protic acid as defined in claim 2, to obtain a solution of the 1:2 (paliperidone:acid) addition salt.

6. The process according to claim 1, wherein the dispersion obtained in step c), or the solution of the 1: 2 addition salt according to claim 5, is treated with a base selected from the group consisting of an alkaline or alkaline earth metal hydroxide, a C₁-C₆ alkoxide and a tertiary amine; preferably sodium or potassium hydroxide.

7. The process according to claim 1, further comprising the subsequent conversion of paliperidone base hydrate to crystalline Form I, by means of a process comprising:
- dissolving paliperidone hydrate in an aqueous solvent as defined in claim 3, or in an organic solvent selected from a straight or branched C₃-C₈ ketone, preferably acetone or methyl ethyl ketone, and a C₁-C₅ alkanol, preferably isopropanol; at a temperature between about 70°C and the solvent boiling point, preferably at about 90°C;
- subsequent cooling of the reaction mixture to a temperature between about 0 and 20°C, preferably to about 0-5°C; and
- recovering the so obtained solid.

8. The process according to claim 7, wherein the solvent is a water/acetone mixture or acetone.

9. The process according to claim 1, further comprising the subsequent conversion of paliperidone base hydrate into crystalline Form I, by means of a process comprising:
- forming a solution of a 1:2 addition salt [paliperidone:acid] with a strong protic acid in water;
- treating said solution with a base dissolved in an organic solvent selected from a C₁-C₅ alkanol and a C₃-C₈ ketone; and
- recovering the so obtained anhydrous paliperidone crystalline Form I.

10. Process according to claim 9, wherein the strong protic acid is as defined in claim 2.

11. The process according to claim 9, wherein the base is as defined in claim 6, preferably triethylamine.

12. The process according to claim 9, wherein the organic solvent is methanol.

13. Crystalline form of paliperidone hydrochloride, having an XRPD spectrum as shown in Figure 1, wherein the most intense diffraction peaks fall at 9.9; 10.3; 11.4; 12.1; 12.7; 14.7; 15.4; 16.1; 19.1; 19.8; 20.9; 24.7; 27.4 ± 0.2° in 2θ.

14. Crystalline form according to claim 13, having a DSC thermogram as shown Figure 2, with a melting peak at 276 ± 2°C.

15. A process for the preparation of paliperidone comprising the alkylation of a compound of formula **(III)** 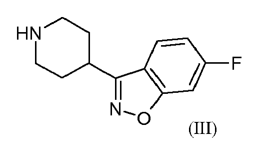 with a compound of formula **(II)** 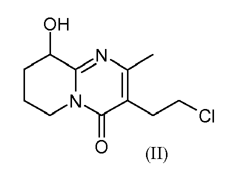 in methanol and in the presence of triethylamine.
